# EUROPEAN PATENT APPLICATION

(11) **EP 4 361 111 A1**
(43) Date of publication of application: **01.05.2024**
(21) Application number: 22204278.0
(22) Date of filing: 28.10.2022
(51) Int. Cl.: C02F 3/32, C12N 1/12

(54) **PROCEDURE FOR OBTAINING MIXED MICROALGAE BACTERIA GRANULES FOR WASTEWATER TREATMENT**

(71) Applicant: National Research and Development Institute for Industrial Ecology - ECOIND, 060652 Bucharest (RO)
(72) Inventor: Tricolici, Olga, Bucharest (RO); Bumbac, Costel, Bucharest (RO); Patroescu, Ion Viorel, Bucharest (RO); Badescu, Valeriu Robert, Bucharest (RO)

(57) **Abstract**

The invention refers to a procedure for obtaining mixed microalgae-bacteria granules for wastewater treatment. The procedure, according to the invention, consists in culturing the microalgae-activated sludge inoculum in a bioreactor by applying the following sequence: feeding the inoculated bioreactor with a liquid medium containing substrate, exposing the bioreactor to a light source, applying a photoperiodicity, mechanical agitation of the medium during the consumption of the substrate, stopping the mechanical stirring, settling the microalgae-activated sludge biomass and medium withdrawn, from which mixed microalgae-activated sludge granules are obtained, with dimensions of 200...3 000 micrometers in diameter and a settling velocity of at least 10 m/h, and which ensures, through gravity sedimentation, the recovery of microalgae from the liquid medium with an efficiency of at least 99%.

## Description

**The invention refers to a procedure** for obtaining a granular structure of the microalgae-activated sludge symbiotic system with high settling capacity, with applications in wastewater treatment.

One of the directions of the sustainable development of socio-ecological complexes is represented by the limited stocks of fossil fuels and the potentially viable solutions for their effective replacement. Microalgal biomass represents a renewable source of energy, which can replace fossil fuel reserves, having numerous advantages over other resources proposed so far [1,2]. Also, other possibilities for the harnessing of microalgal biomass are known: application in the form of fertilizers in agrosystems or nutritional additives in pisciculture, obtaining bioactive compounds (used in the pharmaceutical industry, cosmetics, etc.) [3].

The interest addressed to the biological wastewater treatment procedure using microalgae was due, in particular, to the benefits that can be obtained from the valorization of microalgae biomass. The possibility of obtaining a high production of microalgae in the absence of the costs necessary for procuring nutrients, which come directly from wastewater sources [4], was one of the reasons for researching the procedure at an international level [5].

Conventional biological wastewater treatment processes are known that involve the use of activated sludge biomass composed of various species of bacteria. The residual activated sludge resulting from the treatment process represents a waste [6] whose management is defective as a result of legislative restrictions, the pressure of economic factors, the risk generated on the state of ecosystems and human health [7, 8]. Currently, activated sludge waste is mainly landfilled on the premises of treatment plants - an ineffective long-term solution [9]. Thus, the proposal of feasible solutions for the valorization of residual sludge represents one of the authentic goals, priority for the operators of treatment plants at the global level [10]. In this sense, the partial replacement of activated sludge with microalgal biomass can increase the number of viable options for harnessing residual biomass resulting from treatment processes. The efforts of researchers and treatment plant operators to reduce treatment costs are also known [11], with mechanical aeration processes being attributed to the highest costs, representing approximately 60 - 65% of the total energy costs [12]. The use of photoautotrophic microalgae in the aerobic treatment step can ensure the elimination of mechanical aeration of the reaction medium, with the possibility of supplying the necessary oxygen through the photosynthesis process, in the presence of light [13]. Also, microalgae represent species with high nutrient removal potential (mainly nitrogen and phosphorus) [14], as well as support the regulation function of ecosystems by sequestering carbon dioxide (CO₂) during the photosynthesis process [15].

The procedures reported so far, applied mainly at the laboratory level, have demonstrated the efficiency of using the microalgae-bacteria system for the treatment of different categories of wastewater (municipal, industrial, etc.) [16, 17]. The disadvantage of these processes is the low efficiency of harvesting microalgal cells from the medium - a current problem, intensively addressed not only in the case of wastewater treatment infrastructure but also in the case of the commercial production system of microalgae biomass [18]. This interest is due to the fact that in the absence of an economically efficient method of microalgae harvesting from the environment, the expansion of the process on a large scale is limited, as also the development of the infrastructure addressed to microalgae biomass harnessing for energy production is compromised [19]. Following the analysis of the economic viability of the used microalgae harvesting procedures (chemical coagulation-flocculation, centrifugation, etc.), it was found that the harvesting step represents between 25% and 60% of the total costs associated with microalgae production [20].

The problem of identifying an optimal harvesting procedure is determined by the microscopic dimensions of microalgae cells, the most common microalgae species used for wastewater treatment having a cell diameter of less than 30 µm [21], giving them a settling velocity of less than 10⁻⁶ m/s [22]. The efficiency of applying the simplistic process of gravity sedimentation is strictly dependent on the size of the species, this option being mostly applicable for the removal of large-sized microalgae (> 70 µm), in the rest of the cases being a long-term partial solution (dozens of hours/days) [23].

The current strategy applied in the field of microalgae biotechnology aims the necessity to develop an economically efficient method for the microalgae cells harvesting [24], since, until now, no universal harvesting procedure has been identified to demonstrate viability on a large scale [18].

Several procedures have been proposed for the efficient harvesting of microalgae cells from the effluent/culture medium, the most frequently applied being centrifugation and chemical coagulation/ flocculation. However, although it is the fastest process for harvesting microalgae cells, centrifugation has the disadvantage of high energy requirements [25]. Also, the procedure proved not efficient for all microalgae species [26] and, due to the high centrifugal force, can cause cell lysis [27]. The procedure based on the use of coagulants/flocculants also presents some disadvantages: it was proven to be economically inefficient [18], has the sensitivity to pH variations [23], the efficiency of the procedure is dependent on the concentration of the coagulant and the concentration of the biomass, certain metal salts can cause cell lysis [28] and also presents the risk of biomass contamination with metals [25].

There are also known other harvesting procedures proposed in order to solve the disadvantages of previously mentioned procedures, such as the immobilization of microalgae on different types of matrices and bio-flocculation. Although the cell immobilization method was initially cataloged as the best alternative for cost-efficient wastewater treatment [28], it presents several disadvantages: requires expenditure for the acquisition of matrices, not all matrices ensure effective immobilization of microalgae cells (some matrices being toxic), compared to systems based on suspended microalgae, presents the risk of decreasing the contact surface between microalgae cells and nutrients, requires additional techniques to separate the microalgae cells from the matrices, the efficiency of the procedure is dependent on the biomass concentration and concentration of adhesion substrate, and, as also, presents the risk of decreasing the capture efficiency of the light photons with the increase of biomass thickness [29, 30]. Bio-flocculation is a biological and economic procedure for microalgae recovery based on the use of species with a high auto-flocculation capacity [31]. The success of the procedure application depends on the used microalgae species, however, not all microalgae taxa could be effective in wastewater treatment. Also, another disadvantage of the procedure is the requirement to control the development of a specific target auto-flocculation specie, which is difficult to achieve mainly on a large scale and in real (non-sterile) operating conditions.

The development of granular structures composed of microalgae and fungi species was proposed as another biological procedure for the same purpose, allowing rapid microalgae harvesting by gravitational sedimentation [32]. The disadvantage of the procedure relies on its sensitivity to pH variations, an increase above 7.5 leading to the loss of the granular structure integrity [33]. Also, as in the case of bioflocculation, the procedure is dependent on the used species of fungi [23].

**The technical problem that the procedure solves, according to the invention,** is to treat aerobically wastewater in the absence of mechanical aeration means, using the granular symbiotic system developed between photoautotrophic microalgae and heterotrophic and nitrifying bacteria, and to efficiently and fast harvest microalgae cells from the effluent /cultivation medium by gravity sedimentation, in the absence of the application of other harvesting procedures or the control of operational parameters, by developing of a granular network of the symbiotic system.

**The procedure for obtaining mixed microalgae-bacteria granules, according to the invention.** The symbiosis between photoautotrophic microalgae and heterotrophic and nitrifying bacteria is created by: the consumption of inorganic compounds (NH₄⁺, NO₃⁻, PO₄³⁻, etc.) and the release of molecular oxygen (O₂) into the reaction medium by photoautotrophic microalgae species, by capturing photons during the photosynthesis process, in the light phase; consumption of O₂ by nitrifying bacteria in the nitrification process and by heterotrophic bacteria in the process of organic matter degradation and the release of carbon dioxide (CO₂); assimilation of CO₂ by photoautotrophic microalgae in the light-independent photosynthetic process phase. Through mutual metabolic support, achieved between photoautotrophic microalgae and heterotrophic and nitrifying bacteria, the assimilation of other nutrients from the reaction medium is also ensured. The result of the symbiotic relationships developed between photoautotrophic microalgae and heterotrophic and nitrifying bacteria is represented by the removal of organic matter and nutrients from wastewater without the need for an external source of oxygen.

The granular structure represents a network composed of filamentous microalgae, non-filamentous microalgae (with a cell diameter less than 30 µm), heterotrophic bacteria, and nitrifying bacteria and is the result of symbiotic relationships, morphological properties of filamentous microalgae, biochemical characteristics of microalgae and bacteria, and pressure exercised on mechanical stirring on biomass. Heterotrophic bacteria and nitrifying bacteria, together with photoautotrophic microalgae, ensure a certain degree of cellular aggregation, as a result of the secretion of extracellular polymers, and filamentous microalgae generate a dense network of filaments, which, under the pressure of biomass stirring, determines the granular structure of the symbiotic system, ensuring the adhesion of all types of microalgae to the structure of the granules.

**The purpose of the invention** is to obtain mixed granules of photoautotrophic microalgae and heterotrophic and autotrophic bacteria with a high settling capacity for the development of novel wastewater treatment processes with high treatment efficiency, low operating costs, and multiple possibilities for residual biomass valorization.

**The procedure, according to the invention, presents the following advantages:**
- obtaining a symbiotic microalgae-activated sludge system, with a granular structure and a high sedimentation capacity;
- obtaining a granular system with high resistance of structural integrity to variations of operating parameters;
- efficient and fast removal of microalgae from effluent/culturing medium by gravity sedimentation, in the absence of the application of other complementary methods;
- removal of organic matter and nutrients from wastewater, under aerobic conditions, in the absence of the mechanical aeration process.

The procedure, according to the invention, offers the possibility of increasing the percentage of harnessing of the residual biomass resulting from the wastewater treatment process.

The procedure, according to the invention, could also be applied in the case of the commercial production of microalgae biomass, by replacing wastewater with specific culture media, ensuring efficient harvesting of microalgae cells.

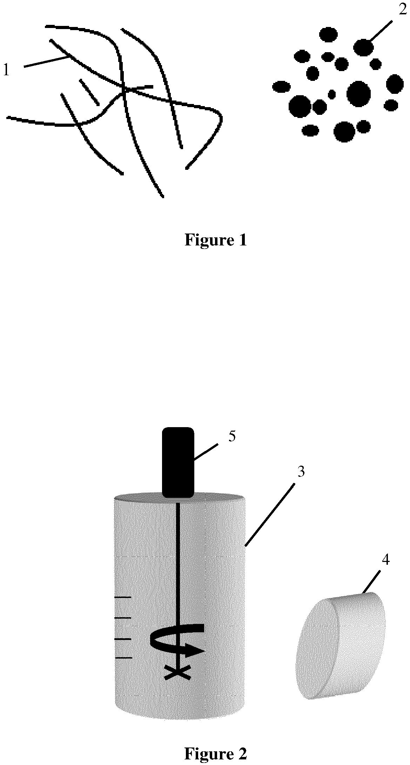

**Two examples of the procedure achievement are presented below, according to the invention,** respectively:
**A.** an example of the accomplishment of the granulation procedure of the symbiotic microalgae-activated sludge system in connection with figures 1, 2, 3, 4, 5, 6, 7, 8, 9, in which:
   - figure 1, represents filamentous microalgae and globular microalgae (non-filamentous),
   - figure 2, illustrates the scheme of the photobioreactor,
   - figure 3, represents the volume distribution of cell size of the non-filamentous microalgae (globular),
   - figure 4, shows the light microscopy image of the symbiotic granular system microalgae-bacteria,
   - figure 5, illustrates the granular structure of the microalgae-bacteria symbiotic system,
   - figure 6, represents the volume distribution of the size of the obtained microalgae-activated sludge granules,
   - figure 7, represents the settling velocity of microalgae-activated sludge granules,
   - figure 8, depicts the settling stage of the microalgae-activated sludge granular entities obtained after the fulfillment of the granulation procedure,
   - figure 9, shows the image of the settling stage of the microalgae-activated sludge symbiotic system captured during the completion of the granulation procedure.
**B.** an example of application of the granular symbiotic system obtained, according to the invention, in connection with figures 10, 11, 12 and table 1, in which:
   - figure 10, represents the capacity of O₂ supply by microalgae and variation of the pH values during the treatment process,
   - figure 11, illustrates the variation of the organic matter concentration (COD),
   - figure 12, represents the variation of ammonium (NH₄⁺), nitrite (NO₂⁻), and nitrate (NO₃⁻) concentrations during treatment process,
   - table 1, represents the physico-chemical characteristics of the influent and effluent resulting from the application of the procedure, according to the invention, in comparison with the regulations in force: NTPA - 001/2005 and NTPA - 002/2005.

**A.** Below is represented an example of the description of the granulation procedure of the symbiotic microalgae-activated sludge system, according to the invention. The procedure of obtaining the granules was carried out in a sequential operating mode with the following steps:
   - photobioreactor inoculation;
   - photobioreactor feeding with 1 liter of wastewater;
   - ensuring the operational parameters: light source, photoperiodicity, the mechanical stirring of the medium, hydraulic retention time;
   - biomass settling for 30 minutes;
   - effluent withdrawn from the upper side of the photobioreactor, without removing the settled biomass, and bioreactor re-feeding.

The granulation procedure of the microalgae-bacteria system was accomplished in a laboratory photobioreactor with a maximum vessel capacity of 2 liters.

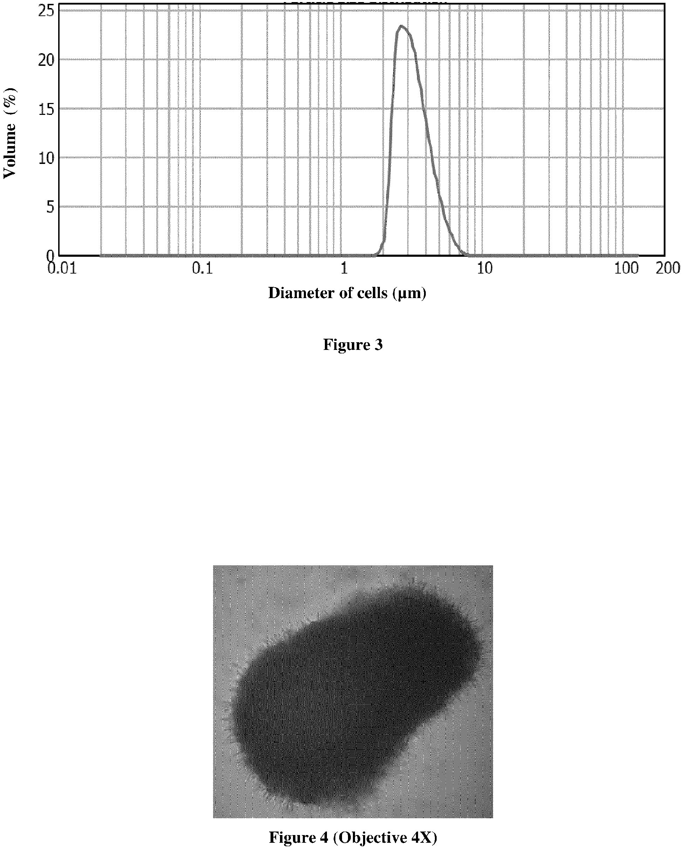

The initial inoculum was represented by native species of filamentous microalgae (1) and non-filamentous (globular) microalgae (2) and heterotrophic and nitrifying bacteria, which developed as a biofilm on the wall of a wastewater treatment reactor. Non-filamentous microalgae were characterized by cell diameter sizes between approximately 1 and 8 µm (Figure 3), having a low sedimentation capacity (< 1.5 - 10⁻⁶ m/s). Filamentous microalgae were characterized by a better sedimentation capacity compared to globular microalgae cells, the value of the indicator being approximately 2.4 . 10⁻⁶ m/s.

The inoculum was pre-enriched in a shaker incubator, equipped with photosynthetic light, in 80% synthetic culture medium enriched in nutrients and 20% wastewater (to which the sampled species were adapted) for a period of one month. During cultivation, the following conditions were set: temperature - 25⁰C, system stirring - 75 rotations/minute (continuous stirring), photoperiodicity frequency: 12 hours light phase: 4 hours dark phase. The stock of microalgae-bacteria culture developed after the cultivation period represented the inoculum used for the granulation procedure.

The influent used to feed the photobioreactor - real dairy wastewater, without autoclaving, had the following physico-chemical characteristics: pH 7...8; O₂ < 30%; COD 100...400 mg O₂/L, NH₄⁺ < 41 mg/L, NO₂⁻ < 0.1 mg/L, NO₃⁻ < 6 mg/L, PO₄³⁻ < 12 mg/L.

The bioreactor (3) was exposed to an external light source, providing an irradiance of 3 980 lumens (4) and the photoperiodicity was set at 15 hours of light phase: 9 hours of the dark phase, the light phase starting during the bioreactor feeding. Biomass homogenization was performed by using a mechanical stirring system (5) of the bioreactor. Parameters: pH, temperature ⁰C, and oxygen saturation % of the reaction medium were monitored continuously by electrodes and sensors embedded in the bioreactor structure. During the procedure, the pH variation was controlled automatically between 6.5 and 8.5 values using potassium hydroxide (KOH) 0.1% and sulfuric acid (H₂SO₄) 1N solutions.

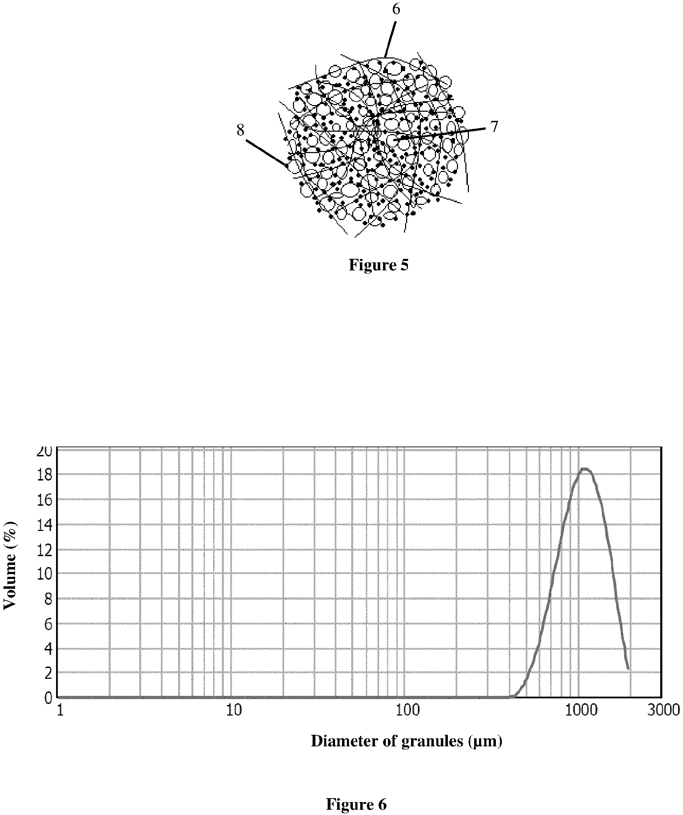

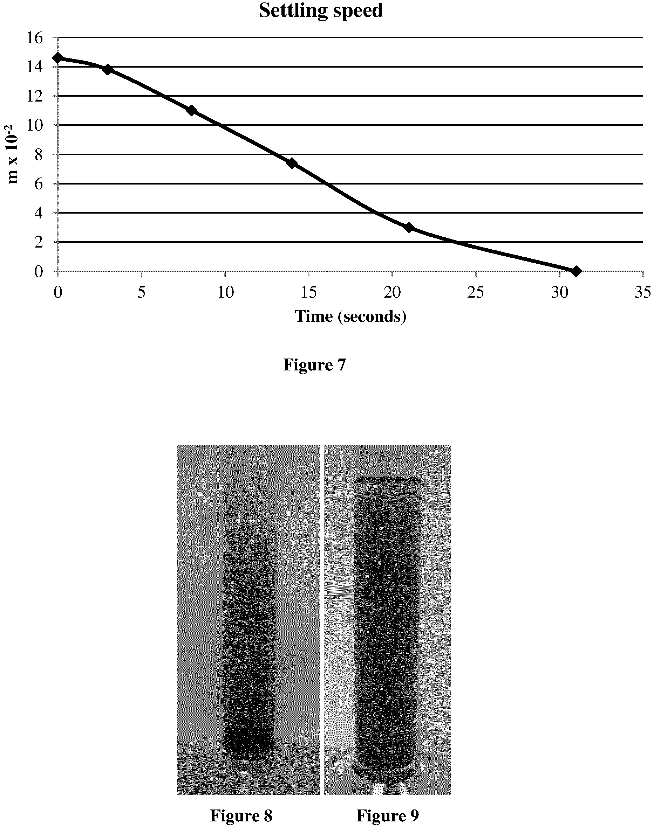

After several operating cycles, a symbiotic granular microalgae-activated sludge system was developed (Figure 4) consisting of filamentous microalgae (6), non-filamentous microalgae (7) and heterotrophic and nitrifying bacteria (8). The resulting granular entities had a diameter ranging between 420 and 1 900 µm (Figure 6), with a sedimentation capacity of about 0.5 . 10' ² ± 0.2 · 10⁻² m/s (Figure 7). Figure 8 shows the image of the microalgae-activated sludge system during the settling stage, at the end of the granulation procedure, and figure 9 shows the image of the microalgae-bacteria system during the settling stage, during the accomplishment of the granulation procedure.

### B. An example of the use of the symbiotic granular microalgae-activated sludge system for wastewater treatment is presented below.

The microalgae-activated sludge inoculum used for wastewater treatment was represented by the granular system obtained by the granulation procedure, according to the invention. The experiment was performed using the type of photobioreactor specified in the granulation procedure. Wastewater treatment was carried out in sequential operation mode in the following steps:
- photobioreactor feeding with pre-treated dairy industry wastewater (1 liter), non-autoclaved, physico-chemical characteristics of the influent are listed in table 1,
- wastewater treatment by applying a hydraulic retention time of 24 hours, with continuous stirring of the biomass (through the mechanical stirring system) at a speed of 120 rotations/minute,
- biomass settling for 5 minutes,
- effluent discharge from the upper part of the bioreactor while maintaining the settled biomass and bioreactor re-feeding.

The bioreactor was exposed to an external light source with an intensity of 3980 lumens, and the photoperiodicity was set to 15 hours of light phase: 9 hours of dark phase, the light phase starting with the feeding of the bioreactor.

According to table 1, the use of the granular microalgae-activated sludge system for wastewater treatment ensures the quality of the effluent, compared to the limits imposed by the NTPA-001/2005 standard, except for the concentration of NO₃⁻ ions.

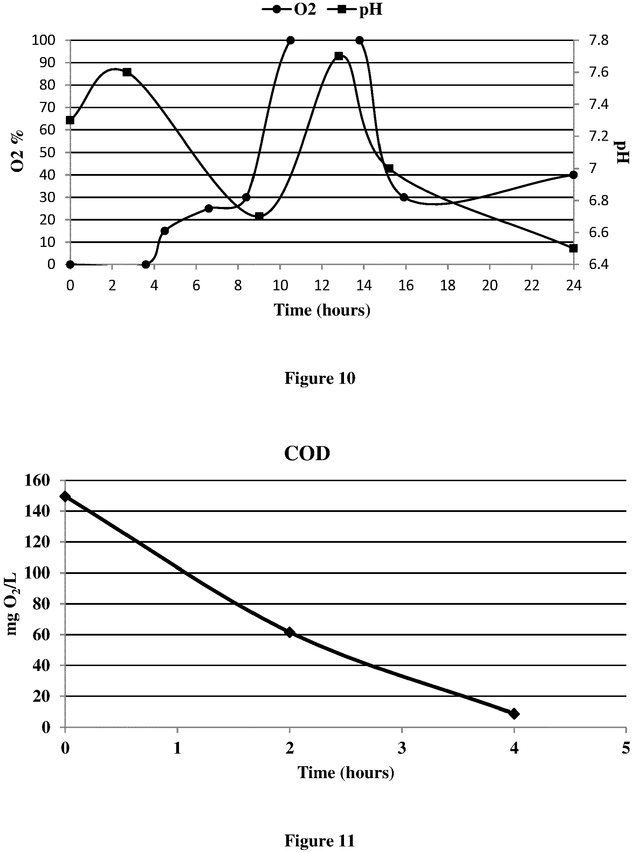

As a result of using the granular microalgae-activated sludge symbiotic system, according to the invention, in the first hours of wastewater treatment, during the light phase, the O₂ saturation level of the reaction medium remained constant at 0% (figure 10), due to the integral consumption of O₂ supply, provided by the process of photosynthesis, by the bacterial biomass. Therefore, during the first hours of treatment, the concentration of molecular oxygen taken over by the bacteria is higher or equal to that provided by the photosynthesis process.

With the decrease in organic matter concentration (COD) from 149.6 to 8.8 mg O₂/L (figure 11), ensured in the first 3-4 hours from the start of the treatment sequence, the O₂ saturation level of the reaction medium began to increase, exceeding the saturation level in molecular oxygen of 100%. Thus, through the biochemical process - of photosynthesis, carried out by the photoautotrophic microalgae, the removal of organic matter was ensured with an efficiency of 94.1%, and the supersaturation of the reaction medium in O₂, in the light phase, in the absence of mechanical aeration.

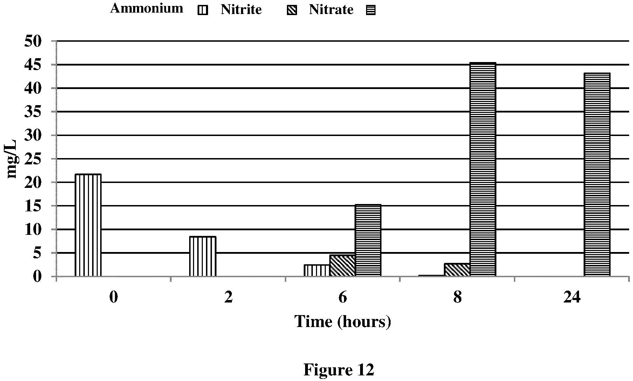

After the almost complete consumption of the organic matter, the initial increase in the O₂ saturation level of the reaction medium is achieved slowly due to the intensification of the nitrification processes (figure 12) along with the increase in the concentration of molecular oxygen in the reaction medium. During the application of the procedure, according to the invention, the removal efficiency of ammonium nitrogen was higher than 99.4%.

Once the entrance into the dark phase, the level of O₂ saturation decreased, reaching a minimum level that varied between 25 and 40%. Variations of O₂ saturation values, during the influent treatment, between 0% and over 100%, did not affect the structural integrity of the granules. During the performing of the procedure, according to the invention, the pH of the reaction medium varied between 6.5 and 7.7 (figure 10) without affecting the structural integrity of the symbiotic microalgae-activated sludge system. The analysis of the effect of shock pressures, represented by variations in pH values between 8.8 and 5.5, highlighted the maintenance of the structural integrity of the symbiotic granular microalgae-activated sludge system.

The harvesting efficiency of microalgae cells was 99.91%, the indicator being assessed based on the chlorophyll "*a*" concentration recorded from biomass, before settling, and from effluent. In the case of other wastewater treatment sequences operated using the granular microalgae-activated sludge symbiotic system, the chlorophyll "*a*" concentration in the effluent ranged between 0 and 0.024 mg/L with a microalgae cells harvesting efficiency between 99.4% and 100%.

### REFERENCES

### Patents

[5] Zhang J., Guo P., Geng J., Ma X., Liu M., 2012. System for cultivating microalgae with wastewater. CN101643699B
[32] Hu Bo, Ruan R. R., Zhang J., Zhou W., 2013. Microalgae culture and harvest. PCT/US2012/059707

### Articles

[1] Pittman K. J., Dean P. A., Osundeko O., 2011. The potential of sustainable algal biofuel production using wastewater resources. Bioresource Technology 102(1), 17-25.
[2] Brennan L., Owende P., 2010. Biofuels from microalgae - A review of technologies for production, processing and extractions of biofuels and co-products. Renewable and Sustainable Energy Reviews 14, 557-577.
[3] Mata M. T., Martins A. A., Caetano S. N., 2010. Microalgae for biodiesel production and other applications: A review. Renewable and Sustainable Energy Reviews 14, 217-232.
[4] Aravantinou A. A., Theodorakopoulos A. M., Manariotis D. I., 2013. Selection of microalgae for wastewater treatment and potential lipids production. Bioresource Technology 147, 130-134.
[8] Perez-Elvira S. I., Diez P. N., Polanco F. F., 2006. Sludge minimisation technologies. Reviews in Environmental Science and Biotechnology 5, 375-398.
[9] AM POS Environment General Directorate, 2012. Elaboration of the national sewage sludge management policy. National sewage sludge management strategy, Part III.
[10] Tyagi K. V., Lo S. -L., 2013. Sludge: A waste or renewable source for energy and resources recovery? Renewable and Sustainable Energy Reviews 25, 708-728.
[11] Leu S. Y., Rosso D. D., Larson L. E., Stenstrom M. K., 2009. Real-time aeration efficiency monitoring in the activated sludge process and methods to reduce energy consumption and operating costs. Water Environment Research 81, 2471-2481.
[12] Fernandez J. F., Castro C. M., Rodrigo A. M., Canizares P., 2011. Reduction of aeration costs by tuning a multi-set point on/off controller: A case study. Control Engineering Practice 19, 1231-1237.
[13] Park J. B. K., Craggs J. R., Shilton N. A., 2011. Wastewater treatment high rate algal ponds for biofuel production. Bioresource Technology 102, 35-42.
[14] Arbib Z., Ruiz J., Alvarez-Diaz P., Garrido-Perez C., Perales J. A., 2013. Capability of different microalgae species for phytoremediation processes: wastewater tertiary treatment, CO2 bio-fixation and low cost biofuels production. Water Research doi: 10.1016/j.watres.2013.10.036.
[15] Razzaka A. S., Hossaina M. M., Lucky A. R., Bassi S. A., de Lasa H., 2013. Integrated CO2 capture, wastewater treatment and biofuel production by microalgae culturing - A review. Renewable and Sustainable Energy Reviews 27, 622-653.
[16] de Godos I., Blanco S., Garcia-Encina A. P., Becares E., Munoz R., 2009. Long-term operation of high rate algal ponds for the bioremediation of piggery wastewaters at high loading rates. Bioresource Technology 100, 4332-4339.
[17] Bhatnagar A., Bhatnagar M., Chinnasamy S., Das K. C., 2010. Chlorella minutissima - A promising fuel alga for cultivation in municipal wastewaters. Applied Biochemistry and Biotechnology 161, 523-536.
[18] Coward T., Lee G. M. J., Caldwell S. G., 2013. Development of a foam flotation system for harvesting microalgae biomass. Algal Research 2, 135-144.
[19] Lee C. -Y., Kim B., Farooq W., Chung J., Han J. -In, Shin H.-J., Jeong H. S., Park Ji - Y., Lee J.-S., Oh Y.-K., 2013. Harvesting of oleaginous Chlorella sp. by organoclays. Bioresource Technology 132, 440-445.
[20] Udom I., Zaribaf H. B., Halfhide T., Gillie B., Dalrymple O., Zhang Q., Ergas J. S., 2013. Harvesting microalgae grown on wastewater. Bioresource Technology 139, 101-106.
[21] Rashid N., Rehman Ur S., Han J. -In, 2013. Rapid harvesting of freshwater microalgae using chitosan. Process Biochemistry 48, 1107-1110.
[22] Granados R. M., Acien G. F., Gomez C., Fernandez-Sevilla M. J., Molina Grima E., 2012. Evaluation of flocculants for the recovery of freshwater microalgae. Bioresource Technology 118, 102-110.
[23] Zhang J., Hu Bo, 2012. A novel method to harvest microalgae via co-culture of filamentous fungi to form cell pellets. Bioresource Technology 114, 529-535.
[24] Pragya N., Pandey K. K., Sahoo K. P., 2013. A review on harvesting, oil extraction and biofuels production technologies from microalgae. Renewable and Sustainable Energy Reviews 24, 159-171.
[25] Grima E. M., Belarbi E. -H., Fernandez A. G. F., Medina R. A., Chisti Y., 2003. Recovery of microalgal biomass and metabolites: process options and economics. Biotechnology Advances 20, 491-515.
[26] Rusten B., Sahu K. A., 2011. Microalgae growth for nutrient recovery from sludge liquor and production of renewable energy. Water Science and Technology 64, 1195-1201.
[27] Papazi A., Makridis P., Divanach P., 2010. Harvesting Chlorella minutissima using cell coagulants. Journal of Applied Phycology 22, 349-355.
[28] Olguin J. E., 2012. Dual purpose microalgae-bacteria-based systems that treat wastewater and produce biodiesel and chemical products within a Biorefinery. Biotechnology Advances 30, 1031-1046.
[29] Moreno-Garrido I., 2008. Microalgae immobilization: current techniques and uses. Bioresource Technology 99, 3949-3964.
[30] Liu K., Li J., Qiao H., Lin A., Wang G., 2012. Immobilization of Chlorella sorokoniana GXNN 01 in alginate for removal of N and P from synthetic wastewater. Bioresource Technology 114, 26-32.
[31] Salim S., Bosma R., Vermue H. M., Wijffels H. R., 2011. Harvesting of microalgae by bio-flocculation. Journal of Applied Phycology 23, 849-855.
[33] Zhou W., Cheng Y., Li Y., Wan Y., Liu Y., Lin X., Ruan R., 2012. Novel fungal pelletization - assisted technology for algae harvesting and wastewater treatment. Applied Biochemistry and Biotechnology 167, 214-228.

### Legislation

[6] Decision no. 856 of August 16, 2002, regarding the record of waste management and for the approval of the list including waste, including hazardous waste.
[7] Order no. 344 of 2004 for the approval of the Technical Norms regarding the protection of the environment and especially of soils, when sewage sludge is used in agriculture.
[34] Normative NTPA-001/2005 regarding the establishment of pollutant loading limits of industrial and urban wastewater when discharged into natural receivers, from Decision no. 352 of April 21, 2005, regarding the amendment and completion of Government Decision no. 188/2002 for the approval of some rules regarding the discharge conditions of wastewater into the aquatic environment.
[35] Normative NTPA-002/2005 regarding the conditions for the discharge of wastewater into the sewerage networks of localities and directly into the treatment plants, from Decision no. 352 of April 21, 2005, regarding the amendment and completion of Government Decision no. 188/2002 for the approval of some rules regarding the discharge conditions of wastewater into the aquatic environment.

**Table 1**

| Parameter | Unit of measurement | Influent | Effluent | NTPA-001/2005 [34] | NTPA-002/2005 [35] |
|---|---|---|---|---|---|
| Temperature | ⁰C | 15 | 28.3 | 35 | 40 |
| pH | - | 7.3 | 6.5 | 6.5-8.5 | 6.5-8.5 |
| O₂ | mg/L | <0.5 | 3.2 | - | - |
| Total suspended matter | mg/L | 4 | 3 | 35¹(60) | 350 |
| Chemical Oxygen Demand - potassium dichromate method (COD) | mg O₂/L | 149.6 | 8.8 | 125 | 500 |
| Biochemical Oxygen Demand at 5 days (BODs) | mg O₂/L | 57.7 | <9 | 25 | 300 |
| N-NH₄+ | mg/L | 16.9 | <0.1 | 2¹ (3) | 30 |
| NO₂⁻ | mg/L | <0.1 | <0.1 | 1¹ (2) | - |
| NO₃⁻ | mg/L | <0.1 | 43.2 | 25¹ (37) | - |
| PO₄³⁻ | mg/L | 9.6 | 4.2 | - | - |
| Total Nitrogen | mg/L | 19.1 | 10.1 | 10¹ (15) | - |
| Total Phosphorus | mg/L | 3.3 | 1.4 | 1¹ (2) | 5 |
| Chlorophyll "*a*" | mg/L | 0 | 20.89/ 0.017² | - | - |

| | | | | | |
|---|---|---|---|---|---|
| ¹ Values to be considered for effluent discharge in sensitive areas ²The concentration of chlorophyll "*a*" of the biomass measured before settling / in effluent | | | | | |

## Claims

1. Procedure for obtaining mixed microalgae-bacteria granules for wastewater treatment, wherein granulation is carried out in photobioreactors operated in sequential operation mode according to the following stages:
**a.** inoculation of the photobioreactor with microalgae and activated sludge;
**b.** feeding the photobioreactor with a liquid medium containing substrate;
**c.** ensuring the operational parameters: exposing the bioreactor to a light source - applying a photoperiodicity; mechanical agitation of the medium to ensure continuous mixing of the liquid, substrate, microalgae, and activated sludge bacteria; operational parameters that provide the simultaneous occurrence of the metabolisms of microalgae and heterotrophic and nitrifying bacteria, in symbiotic relationships, which lead to the transformation of the substrate and the generation of new microalgae and bacterial cells that aggregate among themselves developing granules;
**d.** stopping the stirring to allow the mixed microalgae-activated sludge granules to settle in the photobioreactor;
**e.** effluent discharge by partially emptying the liquid from the top of the photobioreactor;
**f.** repeating the sequence starting from point b.) to point f.).

2. Procedure according to to claim 1, wherein the inoculum described in point a.) is represented by microalgae and bacteria.

3. Procedure according to claim 1, wherein the granulation occurs under the conditions of feeding the photobioreactor in stage b.) with (i) a synthetic liquid medium with organic substrate nutrients, macronutrients, and micronutrients, (ii) real wastewater, or (iii) a mixture of synthetic medium and wastewater which constitutes food for the microorganisms from the structure of the granules.

4. Procedure according to claim 1, wherein the granulation takes place under the conditions of ensuring the operational parameters described at point c.), respectively: external light source, photoperiodicity of 15 hours light phase: 12 hours dark phase, the hydraulic retention time of 24 hours, the light phase starting with the feeding of the bioreactor, mechanical agitation (stirring) at a speed of 120 rpm, pH value control in the range of 6.5...8.5.

5. Procedure according to claim 1, wherein the duration of the settling stage in step d.) progressively decreases from 30 min to 5 min, constituting a selection factor for granular biomass with settling velocity ranging between 10 and 30 m/h.

6. Procedure according to claim 1, wherein the repeated application of steps from point a.) to point f.) leads to obtaining mixed microalgae-activated sludge granules with diameters higher than 420 micrometers.

7. Procedure according to claim 1, wherein the steps from point b.) to point f.) are repeated cyclically until the granular structure is obtained.

8. Procedure according to claim 7, wherein the number of cycles required for complete granulation can be 60 cycles, more than 60 cycles, or less based on the used microalgae species, substrate or wastewater treatment performance.

9. Procedure according to claim 1, wherein activated sludge inoculum can be avoided in accordance with the intended purpose of the microalgae biomass use.

10. Procedure according to claim 1, wherein in none of the stages from point a.) to point f.), no air is introduced from an external source, the oxygen required for biological processes being generated exclusively by the microalgae from the composition of the mixed granules by photosynthesis.

11. Procedure according to claim 10, wherein the obtained granules ensure, by applying the gravitational sedimentation method, under the conditions of a settling time of 5 min, the harvesting of microalgal cells from the medium with an efficiency between 99 and 100%.

12. Procedure according to claim 10, wherein obtained granules can be used for developing novel wastewater treatment processes with the assurance of efficiencies of over 90% for the removal of organic matter and ammonium ions in the conditions of applying a 24 hours hydraulic retention time.

13. Procedure according to claim 4, wherein operational parameters: photoperiodicity, stirring speed, light irradiance and hydraulic retention time can be adjusted based on the used microalgae species, wastewater treatment performance, or in accordance with biomass harness.

14. Granules, according to claim 12, wherein can be used to develop new aerobic treatment processes that do not require an external air source.
